# FASCICULE DE BREVET EUROPEEN

(11) **EP 0 743 993 B1**
(45) Date de publication et mention de la délivrance du brevet: **14.07.1999**
(21) Numéro de dépôt: 95908997.0
(22) Date de dépôt: 08.02.1995
(51) Int. Cl.: D06N 3/04, D06N 3/00, B32B 27/12, A61F 13/15

(54) **MATERIAU NON-TISSE ENDUIT, SON PROCEDE D'OBTENTION ET SON UTILISATION DANS UN ARTICLE D'HYGIENE ABSORBANT JETABLE**
BESCHICHTETES VLIESMATERIAL, VERFAHREN ZU SEINER HERSTELLUNG UND VERWENDUNG IN EINEM ABSORBIERENDEN, HYGIENISCHEN WEGWERFARTIKEL
COATED NONWOVEN MATERIAL, METHOD FOR PREPARING SAME AND USE OF SAID MATERIAL IN A DISPOSABLE ABSORBENT HYGIENE ARTICLE

(30) Priorité: 09.02.1994 FR 9401659
(43) Date de publication de la demande: 27.11.1996
(73) Titulaire: SCA HYGIENE PRODUCTS SA, 59126 Linselles (FR); ELF ATOCHEM S.A., 92800 Puteaux, Hauts-de-Seine (FR); Corovin GmbH, 31201 Peine (DE)
(72) Inventeur: KOCZAB, Jean-Pierre, F-59910 Bondues (FR); DEGRAND, Michel 18, rue Barbey-d'Aurevilly, F-27300 Bernay (FR); DEMESSANCE, Jean, F-77420 Champs-sur-Marne (FR)
(74) Mandataire: Casalonga, Axel
(86) Numéro de dépôt international: FR9500149
(87) Numéro de publication internationale: WO9521957

(56) Documents cités:
- EP-A- 0 187 725
- EP-A- 0 460 375
- DE-A- 2 212 699
- GB-A- 892 799
- DATABASE WPI Week 3875 Derwent Publications Ltd., London, GB; AN 75-63022W (38) & JP,A,50 034 376 (NIPPON UNICAR CO) , 2 Avril 1975
- DATABASE WPI Section Ch, Week 9030 Derwent Publications Ltd., London, GB; Class A96, AN 90-227856 & JP,A,02 155 639 ( CHISSO CORP) , 14 Juin 1990

## Description

La présente invention se rapporte à un matériau non-tissé enduit comprenant une couche de base formée d'une nappe non-tissée en matériau fibreux et au moins une couche d'un film thermoplastique appliqué à chaud sur une des faces de ladite couche de base. Ce matériau non-tissé enduit est utilisable notamment dans les articles d'hygiène absorbants jetables tels que les couches-culottes pour bébés ou pour incontinents ainsi que les culottes d'entraînement à la propreté et les serviettes périodiques, comprenant une feuille extérieure de support imperméable aux liquides, une feuille intérieure de couverture perméable aux liquides et un coussin absorbant disposé entre lesdites feuilles et fixé au moins sur la face interne de la feuille de support, pour remplacer notamment ladite feuille extérieure de support qui est constituée habituellement d'un film mince de polyéthylène, afin de lui donner un aspect et un toucher textile plus agréable pour l'utilisateur.

L'invention concerne également un procédé pour obtenir ledit matériau non-tissé enduit.

Il est connu dans l'état de la technique de procéder à l'enduction de matériaux fibreux non-tissés avec un film thermoplastique. Un tel matériau est décrit dans le brevet GB 1 403 603. Cependant, on ne donne dans ce brevet aucune indication sur l'adhérence entre les deux couches, ni sur l'aspect textile du produit obtenu.

Le brevet EP 0 187 725 décrit une étoffe revêtue comprenant une couche de base en un matériau en fibres non-tissé dans laquelle sont préformées des parties densifiées et non densifiées et un film thermoplastique lié à chaud à une face de ladite couche de base de façon à ce que la profondeur de pénétration du film dans ladite couche de base soit limitée à moins de l'épaisseur totale de cette dernière.

Selon ce brevet, cette pénétration du film thermoplastique dans ladite couche de base est nécessaire pour obtenir une meilleure adhérence du film thermoplastique à la couche de base en matériau en fibres non-tissées.

Compte tenu de l'épaisseur importante dudit film thermoplastique (celle-ci pouvant varier de 10 à 250 microns), la pénétration dudit film dans le matériau en fibres non-tissées, favorisée par la température et la pression lors de la mise en oeuvre, devient un inconvénient majeur du fait qu'une quantité non négligeable du film thermoplastique fondu peut pénétrer dans les zones non densifiées du matériau en fibres non-tissées, entraînant ainsi une diminution du toucher et de la douceur semblable à celle d'un tissu de la face dudit matériau fibreux non enduit.

Le document JP-2-155639 décrit un matériau non-tissé enduit ayant une couche de base comportant au moins 30 % en poids de fibres composites thermo-adhésives et un film poreux, appliqué par pressage à chaud sur la couche de base à une température supérieure à la température de thermo-adhésion des fibres composites au moyen d'un cylindre d'embossage ayant 5 à 40 % de zones convexes.

On a maintenant trouvé un matériau non-tissé enduit ayant une souplesse et un toucher textile améliorés, comprenant une couche de base formée d'une nappe non-tissée en matériau fibreux et au moins une couche d'un film thermoplastique appliqué sur la couche de base et comprenant au moins un polymère d'éthylène, caractérisé en ce que le film thermoplastique comprend au moins un agent antibloquant, adhère uniformément seulement à la surface de la couche de base et a une résistance au pelage, déterminée selon la norme NFT 76-112 (conditions : angle de pelage 180°, vitesse de traction 200 mm/mn), au plus égale à 98.10⁻³ kN/m et de préférence comprise entre 6.10⁻³ et 70.10⁻³ kN/m.

Selon la présente invention, de préférence le film thermoplastique en contact avec la couche de base formée d'une nappe non-tissée en matériau fibreux a une épaisseur au plus égale à 10µm (microns) et, de préférence, une épaisseur allant de 5 µm à 8 µm.

Selon une réalisation préférée, le film thermoplastique comprend au moins un polymère d'éthylène associé avec au moins un agent antibloquant.

Le polymère d'éthylène peut être choisi dans le groupe comprenant les copolymères d'éthylène et d'au moins une α-oléfine ayant de 3 à 8 atomes de carbone, les copolymères éthylène / (méth)acrylate d'alkyle dans lesquels le groupement alkyl peut être linéaire ou ramifié ayant un nombre de carbone allant de 1 à 6, les copolymères d'éthylène et d'au moins un ester vinylique d'acide carboxylique saturé ayant de 2 à 6 atomes de carbone.

A titre d'illustration de polymère d'éthylène, on citera le polyéthylène base densité, le polyéthylène très base densité, les copolymères éthylène/(méth)acrylate de méthyle, les copolymères éthylène/(méth)acrylate d'éthyle, les copolymères éthylène/(méth) acrylate de butyle, les copolymères éthylène/acétate de vinyle et le mélange d'au moins deux des copolymères précités.

Parmi ces polymères d'éthylène, l'invention concerne de préférence les copolymères éthylène/(méth)acrylate d'alkyle et plus particulièrement les copolymères éthylène/(méth)acrylate de méthyle.

Selon la présente invention, de préférence les copolymères éthylène/(méth)acrylate d'alkyle comprennent de 2,5% à 40% en poids, de préférence de 15% à 30% en poids d'unités dérivées de (méth) acrylate d'alkyle. Ils présentent un indice de fluidité exprimé en g/10 mn déterminé selon la norme ISO 1133 (conditions : 190°C, charge 2,16 kg) au moins égal à 0,5 et de préférence compris entre 3 et 10, et possèdent une densité exprimée en g/cm³ déterminée selon la norme ISO 1183 allant de 0,91 à 0,96.

L'agent anti-bloquant peut être choisi dans le groupe comprenant des charges minérales, des amides d'acide gras insaturés ayant au moins 8 atomes de carbone, des éthylène-bis amides d'acide gras insaturés ayant au moins 8 atomes de carbone.

A titre d'illustration de tels agents anti-bloquants, on citera la silice, le talc, le stéarate de zinc, les amides stéarique, oléique, palmitique, érucique, myristique, béhénique, l'éthylène-bis-oléamide, l'éthylène-bis-érucamide, l'éthylène-bis-stéaramide.

De préférence, on utilisera le talc et/ou l'éthylène-bis-stéaramide.

Ces agents anti-bloquants selon la présente invention sont utilisés à des teneurs allant de 0,01 à 2% en poids et de préférence 0,1 à 1,5% d'agent anti-bloquant par rapport au polymère d'éthylène.

Selon la présente invention, la nappe non-tissée en matériau fibreux peut être fabriquée par le procédé de liaison au filage ("spun bonded") et thermoliage, à partir de filaments à orientation moléculaire, essentiellement continus, à base de polypropylène(homo-polymère) et de grammage compris entre 14 g/m² et 30 g/m²; de préférence les filaments ont un diamètre compris entre 1,8 et 2,2 dtex; en outre, l'adjonction d'un additif tel qu'une (N-alkyl)amide d'acide gras à raison de 0,8 à 1% en poids par rapport à la base thermoplastique améliore la douceur et les caractéristiques textiles de la nappe non-tissée.

Selon la forme des motifs de thermoliage et le pourcentage de points de liage de cette nappe non-tissée, on obtient un matériau ayant une plus ou moins grande élasticité et douceur.

Des nappes non-tissées obtenues par tout autre procédé de fabrication tel que par laçage au filage (spun lace) ou enchevêtrement par jets d'eau, liaison chimique, liaison thermique, aiguilletage, soufflage-fusion (melt blown), nappage par flux d'air (air laid) ou des nappes composites associant plusieurs couches différentes obtenues par au moins deux desdits procédés précédents, peuvent être aussi utilisées.

D'autres types de fibres synthétiques, par exemple à base de polyéthylène, de polyester, ainsi que des mélanges de celles-ci, de même deniers ou de deniers différents, peuvent être employés.

On peut également utiliser des non-tissés "spun" contenant un copolymère d'éthylène/acrylate d'alkyle à faible point de fusion favorisant l'adhésion de surface.

Un autre objet de l'invention concerne également l'utilisation d'un tel matériau non-tissé enduit, dans un article d'hygiène absorbant jetable tel qu'une couche-culotte ou une culotte d'entraînement à la propreté, comme feuille extérieure de support imperméable aux liquides, la couche de base formée d'une nappe fibreuse étant disposée vers l'extérieur de la couche-culotte.

Dans un autre mode d'utilisation recommandé, dans un article d'hygiène absorbant jetable tel qu'une couche-culotte, le matériau non-tissé enduit peut être utilisé comme feuille intérieure de couverture pourvue d'une ouverture centrale et d'éléments élastiques coopérant avec cette dernière pour faciliter le passage de l'urine et des selles et leur isolation de la peau de l'utilisateur; dans ce cas, la couche de base formée d'une nappe fibreuse est disposée de façon à former la face externe venant en contact avec la peau de l'utilisateur.

Egalement, de préférence, le matériau non-tissé enduit peut être utilisé pour former des volets ou barrières longitudinales d'étanchéité élastifiées fixés au-dessus ou le long des bords longitudinaux du coussin absorbant à la feuille intérieure de couverture de la couche-culotte, qui viennent en contact avec les cuisses de l'utilisateur et empêchent les fuites transversales en direction des bords longitudinaux de ladite couche-culotte; la couche de base formée d'une nappe fibreuse est aussi disposée de façon à former la face externe venant en contact avec la peau de l'utilisateur.

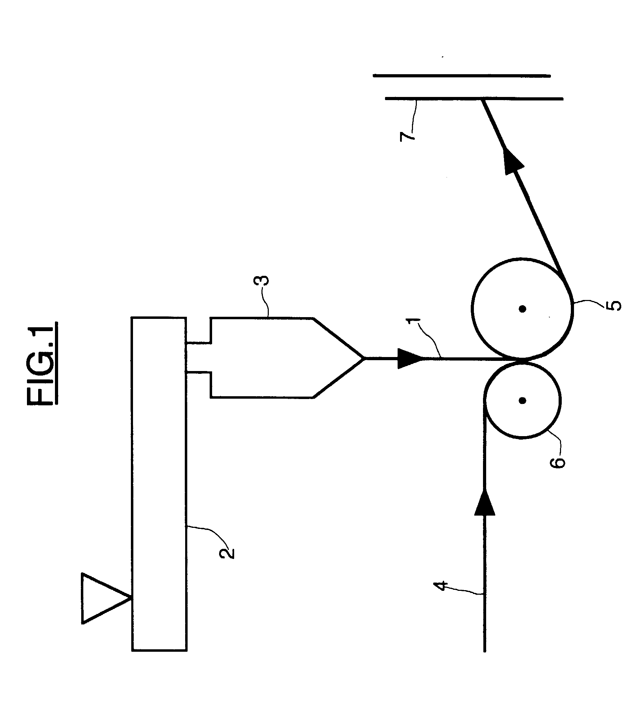

Le matériau non-tissé enduit selon la présente invention, peut être obtenu selon un procédé schématisé sur la figure 1 qui consiste à réaliser :
- Dans une première étape, connue en soi, l'extrusion d'un film thermoplastique (1) à partir d'au moins une extrudeuse (2) possédant une filière (3) à entrefer réglable.

Cette extrudeuse est alimentée avec un mélange comprenant au moins un polymère d'éthylène et au moins un agent anti-bloquant, ledit mélange pouvant éventuellement contenir des additifs classiques tels que pigments, colorants, stabilisants.

La température matière en sortie de filière peut être comprise entre 280°C et 320°C.
- Dans une seconde étape, le film thermoplastique (1) extrudé, présentant une température au plus égale à 320°C, non encore solidifié, est mis en contact (appliqué) avec une bande de non-tissé (4). Cette étape d'enduction couchage est réalisée au moyen de deux cylindres (5) et (6) entre lesquels sont amenés la bande de non-tissé (4) et le film thermoplastique (1). Le cylindre (5) en contact direct avec le film thermoplastique, désigné généralement par "chill roll", peut être refroidi à l'eau à une température au plus égale à 25°C et, de préférence, comprise entre 15°C et 20°C. Sa surface peut être brillante, mate ou éventuellement grainée.

Selon la présente invention, l'agent anti-bloquant permet d'atténuer très fortement l'adhésion du film thermoplastique sur le chill roll.

On ne sortirait pas du cadre de l'invention, si la surface du "chill roll" était revêtue d'un revêtement anti-adhérent tel qu'un revêtement en Téflon.

Le cylindre presseur (6), de diamètre généralement inférieur, peut être également refroidi.

La pression entre les deux cylindres peut être au plus égale à 6.10⁵ Pa et, de préférence, comprise entre 2 et 5.10⁵ Pa.

Selon un mode préféré de l'invention, la distance entre la sortie de la filière et le point de contact du film plastique avec la bande de non-tissé (distance généralement désignée par le terme anglais "air gap") est au plus égale à 200 mm et, de préférence, comprise entre 50 et 150 mm.
- Dans une troisième étape, la bande de non-tissé revêtue du film thermoplastique est entraînée par un banc de tirage (7) dont la vitesse est au plus égale à 300 m/mn et de préférence comprise entre 100 et 150 m/mn, la tension de tirage est faible et parfaitement contrôlée pour éviter toute déformation de la bande de non-tissé revêtue du film thermoplastique. Elle est au plus égale à 5.10⁵ Pa, et de préférence comprise entre 2 et 3.10⁵ Pa. Ensuite, après avoir subi éventuellement une opération d'impression en ligne sur la face externe du film, le matériau est enroulé sous faible tension.

En réglant la température d'extrusion, le débit, l'entrefer, l"'air gap", la pression entre les cylindres et leur température, la vitesse et la tension de tirage, on obtient un matériau non-tissé revêtu d'un film thermoplastique de faible épaisseur; néanmoins, cette faible épaisseur n'affecte pas les propriétés d'imperméabilité aux liquides du matériau obtenu.

Ce film adhére seulement sur la surface du non-tissé de façon uniforme.

La résistance au pelage est faible, égale ou inférieure à 98.10⁻³ kN/m; mais suffisante pour assurer une processabilité pour les utilisations envisagées. Ce mode d'accrochage permet d'obtenir un matériau présentant une grande souplesse et est peu bruyant au froissement. Il présente également l'avantage de conserver le toucher initial du non-tissé, en particulier le toucher textile.

L'exemple suivant illustre l'invention.

On utilise une extrudeuse monovis présentant un diamètre D égal à 60 mm et une longueur de 30 D.

Cette extrudeuse est alimentée par un mélange constitué de :
- 100 parties en poids d'un mélange comprenant
   . 98,3% en poids d'un copolymère éthylène/acrylate de méthyle contenant 20% en poids d'unité dérivée d'acrylate de méthyle et 80% en poids d'unité dérivée d'éthylène, présentant un indice de fluidité égal à 8 g/10 mn, mesuré selon la norme ISO 1133 (conditions : 190°C, charge 2,16 kg),
   . 0,2% en poids d'éthylène-bis-stéaramide, et
   . 1,5% en poids de talc,
   commercialisé par la Société ELF ATOCHEM S.A., sous la dénomination "LOTRYL 20 MB 08", et de
- 7 parties en poids d'un mélange maître à base de polyéthylène contenant 60% en poids de TiO₂.

L'extrusion est réalisée à une température comprise entre 240°C et 280°C avec une vitesse de vis égale à 50 tr/mn et un débit de 50 kg/h. La filière a une longueur utile égale à 900 mm et une ouverture d'entrefer de 400 microns.

Le film sortant de la filière est à 280°C et est appliqué vers les 2 cylindres (5) et (6) pour être déposé sur une bande de non-tissé en polypropylène de grammage égal à 16 g/m², commercialisé par la Société COROVIN GmbH sous la dénomination "CORONOVO DOUCE", la surface des zones thermoliées de ladite bande de non-tissé a une valeur nominale de 6,88% et se compose de 16 points de liage au cm².

L"'air gap" est de 150 mm.

Le "chill roll" (5) est refroidi par de l'eau à 18°C.

La pression appliquée par le cylindre presseur (6) est de 2.10⁵ Pa.

La vitesse de ligne est de 100 m/mn.

La tension de tirage est d'environ 3.10⁵ Pa.

Le matériau non-tissé, enduit avec un film thermoplastique, obtenu, est coupé puis enroulé.

Sur un prélèvement du matériau ainsi obtenu, on réalise une mesure de résistance au pelage selon la norme NFT 76-112 (mai 1982) selon les conditions : angle de pelage égal à 180°, vitesse de traction 200 mm/mn.

Cette résistance au pelage est de 15.10⁻³ kN/m pour un grammage de 9,5 g/m² du film; un autre exemple d'enduction d'un non-tissé de 20 g/m² par un film de 13 g/m² a donné une valeur de résistance au pelage de 70.10⁻³ kN/m.

L'observation au microscope électronique à balayage (MEB) ainsi qu'au stéréomicroscope avec une lumière incidente rasante des composants du matériau après délamination, montre que l'adhésion se fait de façon uniforme sur la surface du non-tissé.

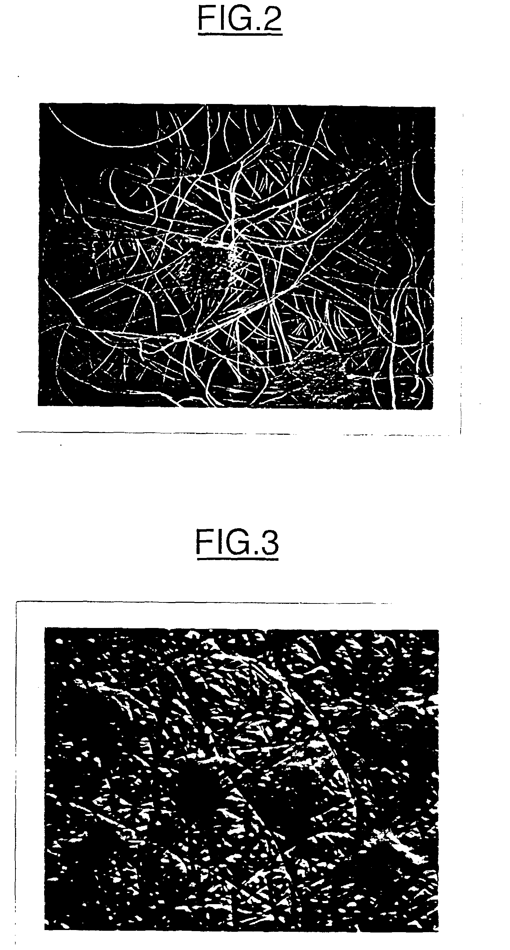

Sur la figure 2, qui est une photographie avec un grossissement de 17 de la surface du non-tissé après délamination, c'est-à-dire après décollage du film, on n'observe plus de trace du film thermoplastique. L'enchevêtrement des fibres libres continues du non-tissé n'a pas été modifié par le décollage du film thermoplastique, ce qui indique une adhérence superficielle entre le film thermoplastique et le non-tissé.

Les zones où les fibres du non-tissé apparaissent localement fondues, correspondent en fait aux zones de liage du non-tissé (zones prédensifiées).

Par contre, sur la figure 3, qui est une photographie obtenue au stéréomicroscope en lumière rasante, après un grossissement de 12 de la surface du film thermoplastique après délamination de la couche de base en non-tissé, on voit nettement l'empreinte des fibres de surface du non-tissé qui ont participé à l'accrochage.

Ceci montre bien que le film thermoplastique forme un enrobage des fibres de surface sans pénétration substantielle dans l'épaisseur du matériau non-tissé.

Il est bien entendu que l'on pourrait envisager l'utilisation du matériau enduit selon l'invention dans de nombreux autres domaines d'application, tels que dans le domaine médical, notamment dans les champs opératoires, les revêtements protecteurs, les pansements et les gants.

## Revendications

1. Matériau non-tissé enduit, comprenant une couche de base formée d'une nappe non-tissée en matériau fibreux et au moins une couche d'un film thermoplastique appliqué sur la couche de base et comprenant au moins un polymère d'éthylène caractérisé en ce que le film thermoplastique comprend au moins un agent anti-bloquant, adhère uniformément seulement à la surface de la couche de base et a une résistance au pelage, déterminée selon la norme NFT 76-112 (condition : angle de pelage 180°, vitesse de traction 200 mm/mn) au plus égale à 98.10⁻³ kN/m.

2. Matériau selon la revendication 1, caractérisé en ce que la résistance au pelage est comprise entre 6.10⁻³ et 70.10⁻³ kN/m.

3. Matériau selon l'une quelconque des revendications 1 ou 2, caractérisé en ce que le film thermoplastique a une épaisseur au plus égale à 10 µm (microns).

4. Matériau selon la revendication 3, caractérisé en ce que le film thermoplastique a une épaisseur allant de 5 à 8 µm (microns).

5. Matériau selon l'une quelconque des revendications 1 à 4, caractérisé en ce que le polymère d'éthylène est choisi dans le groupe comprenant des copolymères de l'éthylène et d'au moins une α-oléfine ayant de 3 à 8 atomes de carbone, les copolymères éthylène/(méth)acrylate d'alkyle dans lesquels le groupement alkyle est linéaire ou ramifié et a un nombre de carbone allant de 1 à 6, les copolymères d'éthylène et d'au moins un ester vinylique d'acide carboxylique saturé ayant de 2 à 6 atomes de carbone.

6. Matériau selon la revendication 5, caractérisé en ce que le polymère d'éthylène est choisi parmi les copolymères éthylène/(méth) acrylate d'alkyle.

7. Matériau selon la revendication 6, caractérisé en ce que le polymère d'éthylène est choisi parmi les copolymères éthylène/(méth)acrylate de méthyle.

8. Matériau selon la revendication 7, caractérisé en ce que le polymère d'éthylène est le copolymère éthylène/acrylate de méthyle.

9. Matériau selon l'une quelconque des revendications 6 à 8, caractérisé en ce que les copolymères éthylène/(méth)acrylate d'alkyle comprennent de 2,5% à 40% en poids d'unités dérivées de (méth)acrylate d'alkyle.

10. Matériau selon l'une quelconque des revendications 1 à 9, caractérisé en ce que l'agent anti-bloquant est choisi dans le groupe comprenant des charges minérales, des amides d'acides gras insaturés ayant au moins 8 atomes de carbone, des éthylène-bis amides d'acides gras insaturés ayant au moins 8 atomes de carbone.

11. Matériau selon la revendication 10, caractérisé en ce que l'agent anti-bloquant est choisi dans le groupe comprenant la silice, le talc, le stéarate de zinc, les amides stéarique, oléique, palmitique, érucique, myristique, béhénique, l'éthylène-bis-oléamide, l'éthylène-bis-érucamide, l'éthylène-bis-stéaramide.

12. Matériau selon ls revendication 11, caractérisé en ce que l'agent anti-bloquant est le talc et/ou l'éthylène-bis-stéaramide.

13. Matériau selon l'une quelconque des revendications 1 à 12, caractérisé en ce que la nappe non-tissée en matériau fibreux est fabriquée par liaison au filage ("spun bonded") et thermoliage, à partir de filaments à orientation moléculaire, essentiellement continus, à base de polypropylène (homopolymère), et a un grammage compris entre 14 g/m² et 30 g/m².

14. Matériau selon la revendication 13, caractérisé en ce que la composition à base de polypropylène est modifiée par l'adjonction de 0,8 à 1% en poids d'une (N-alkyl)amide d'acide gras et que les filaments ont un diamètre compris entre 1,8 et 2,2 dtex.

15. Procédé de fabrication d'un matériau non-tissé enduit selon l'une quelconque des revendications 1 à 14, qui consiste à réaliser :
- l'extrusion d'un film thermoplastique (1),
- la mise en contact dudit film thermoplastique avec une bande de non-tissé (4), et
- l'entraînement de la bande de non-tissé revêtu dudit film thermoplastique;
caractérisé en ce que la mise en contact du film thermoplastique extrudé (1), présentant une température au plus égale à 320°C non encore solidifié, avec la bande de non-tissé (4), est réalisée au moyen de deux cylindres (5, 6) entre lesquels la pression est au plus égale à 6.10⁵ Pa, le cylindre (5) en contact direct avec le film thermoplastique extrudé (1) étant à une température au plus égale à 25°C et que l'entraînement de la bande de non-tissé revêtu du film thermoplastique est réalisé par un banc de tirage dont la vitesse est au plus égale à 300 m/mn et exerçant une tension de tirage au plus égale à 5.10⁵ Pa.

16. Article d'hygiène absorbant jetable tel qu'une couche-culotte ou une culotte d'entraînement à la propreté, caractérisé en ce que la feuille de support imperméable aux liquides est constituée par le matériau selon l'une quelconque des revendications 1 à 14, avec la couche de base formée d'une nappe fibreuse disposée vers l'extérieur de la couche-culotte.

17. Article d'hygiène absorbant jetable tel qu'une couche-culotte dans laquelle la feuille intérieure de couverture est pourvue d'une ouverture centrale et d'éléments élastiques coopérant avec cette dernière, caractérisé en ce que ladite feuille est constituée par le matériau selon l'une quelconque des revendications 1 à 14, avec la couche de base formée d'une nappe fibreuse disposée de façon à former la face externe venant en contact avec la peau de l'utilisateur.

18. Article d'hygiène absorbant jetable tel qu'une couche-culotte comprenant des volets ou barrières longitudinales d'étanchéité élastifiées, fixés au-dessus ou le long des bords longitudinaux du coussin absorbant à la feuille intérieure de couverture de ladite couche-culotte, caractérisé en ce que lesdits volets sont constitués par le matériau selon l'une quelconque des revendications 1 à 14, avec la couche de base formée d'une nappe fibreuse disposée de façon à former la face externe venant en contact avec la peau de l'utilisateur.

## Patentansprüche

1. Beschichtetes Vliesmaterial, umfassend eine Basisschicht aus einer Vliesschicht aus Fasermaterial und mindestens eine Schicht eines thermoplastischen Films, der auf die Basisschicht aufgebracht ist und mindestens ein Ethylen-Polymer enthält,
dadurch **gekennzeichnet,** daß
der thermoplastische Film mindestens ein Antiblockungsmittel enthält, einheitlich allein auf der Oberfläche der Basisschicht haftet und einen Schälwiderstand, bestimmt gemäß der Norm NFT 76-112 (Bedingungen: Schälwinkel = 180°, Ziehgeschwindigkeit = 200 mm/min), von höchstens 98 x 10⁻³ kN/m aufweist.

2. Material gemäß Anspruch 1,
dadurch **gekennzeichnet,** daß
der Schälwiderstand 6 bis 70 x 10⁻³ kN/m beträgt.

3. Material gemäß einem der Ansprüche 1 oder 2,
dadurch **gekennzeichnet,** daß
der thermoplastische Film eine Dicke von höchstens 10 µm aufweist.

4. Material gemäß Anspruch 3,
dadurch **gekennzeichnet,** daß
der thermoplastische Film eine Dicke von 5 bis 8 µm aufweist.

5. Material gemäß einem der Ansprüche 1 bis 4,
dadurch **gekennzeichnet,** daß
das Ethylen-Polymer aus der Gruppe ausgewählt ist, umfassend Copolymere aus Ethylen und mindestens einem α-Olefin mit 3 bis 8 Kohlenstoffatomen, Ethylen/Alkyl (meth)acrylat-Copolymere, in denen die Alkylgruppe linear oder verzweigt ist und eine Kohlenstoffzahl von 1 bis 6 aufweist, und Copolymere aus Ethylen und mindestens einem gesättigten Carboxylsäurevinylester mit 2 bis 6 Kohlenstoffatomen.

6. Material gemäß Anpruch 5,
dadurch **gekennzeichnet,** daß
das Ethylen-Polymer aus Ethylen/Alkyl(meth)acrylat-Copolymeren ausgewählt ist.

7. Material gemäß Anspruch 6,
dadurch **gekennzeichnet,** daß
das Ethylen-Polymer aus Ethylen/Methyl(meth)acrylat-Copolymeren ausgewählt ist.

8. Material gemäß Anspruch 7,
dadurch **gekennzeichnet,** daß
das Ethylen-Polmer ein Ethylen/Methylacrylat-Copolymer ist.

9. Material gemäß einem der Ansprüche 6 bis 8,
dadurch **gekennzeichnet,** daß
die Ethylen/Alkyl(meth)acrylat-Copolymeren 2,5 bis 40 Gew.% Alkyl (meth)acrylat-Einheiten enthalten.

10. Material gemäß einem der Ansprüche 1 bis 9,
dadurch **gekennzeichnet,** daß
das Antiblockungsmittel aus der Gruppe ausgewählt ist, umfassend mineralische Beaufschlagungsmittel, ungesättigte Fettsäureamide mit mindestens 8 Kohlenstoffatomen und Ethylenbisamide ungesättigter Fettsäuren mit mindestens 8 Kohlenstoffatomen.

11. Material gemäß Ansporuch 10,
dadurch **gekennzeichnet,** daß
das Antiblockungsmittel aus der Gruppe, umfassend Kieselsäure, Talkum, Zinkstearat, Stearin-, Olein-, Palmitin-, Erucin-, Myristin-, Behenamide, Ethylenbisoleamid, Ethylenbiserucamid und Ethylenbisstearamid, ausgewählt ist.

12. Material gemäß Anspruch 11,
dadurch **gekennzeichnet,** daß
das Antiblockungsmittel Talkum und/oder Ethylenbisstearamid ist.

13. Material gemäß einem der Ansprüche 1 bis 12,
dadurch **gekennzeichnet,** daß
die Vliesschicht aus Fasermaterial durch Spinnen ("spun bonded") und Thermobindung, ausgehend von im wesentlichen kontinuierlichen Filamenten mit molekularer Orientierung auf Basis von Polypropylen (Homopolymer), hergestellt ist und ein Grammgewicht von 14 bis 30 g/m² aufweist.

14. Material gemäß Anspruch 13,
dadurch **gekennzeichnet,** daß
die Zusammensetzung auf Basis von Polypropylen durch Zufügung von 0,8 bis 1 Gew.% (N-Alkyl)fettsäureamid modifiziert ist und die Filamente einen Durchmesser von 1,8 bis 2,2 dtex aufweisen.

15. Verfahren zur Herstellung eines beschichteten Vliesmaterials gemäß einem der Ansprüche 1 bis 14, welches darauf beruht, daß man:
- einen thermoplastischen Film (1) extrudiert,
- den genannten thermoplastischen Film mit einer Bahn (4) aus Vliesstoff in Kontakt bringt, und man
- die mit dem genannten thermoplastischen Film überzogene Bahn aus Vliesstoff wegzieht,
dadurch **gekennzeichnet,** daß
der extrudierte thermoplastische Film (1), der eine Temperatur von höchstens 320°C aufweist und noch nicht fest geworden ist, mit der Bahn (4) aus Vliesstoff mittels zweier Zylinder (5), (6) in Kontakt gebracht wird, zwischen denen der Pressdruck höchstens 6 x 10⁵ Pa beträgt, wobei der Zylinder (5) in direktem Kontakt mit dem extrudierten thermoplastischen Film (1) eine Temperatur von höchstens 25°C aufweist, und das Wegziehen der mit dem thermoplastischen Film überzogenen Bahn aus Vliesstoff mit einer Ziehbank durchgeführt wird, deren Ziehgeschwindigkeit höchstens 300 m/min und deren ausgeübte Zugspannung höchstens 5 x 10⁵ Pa betragen.

16. Absorbierender verwerfbarer Hygieneartikel wie ein Einlagen- bzw. Windelhöschen oder ein Höschen zur persönlichen Reinhaltung,
dadurch **gekennzeichnet,** daß
die für Flüssigkeiten undurchlässige Trägerunterlage aus dem Material gemäß einem der Ansprüche 1 bis 14 zusammengesetzt ist, worin die aus einer Faserschicht gebildete Basisschicht auf der Aussenseite des Einlagen- bzw. Windelhöschens angeordnet ist.

17. Absorbierender verwerfbarer Hygieneartikel wie ein Einlagen- bzw. Windelhöschen, worin die innere Deckschicht mit einer zentralen Öffnung und elastischen Elementen versehen ist, die mit der letzteren zusammenwirken,
dadurch **gekennzeichnet,** daß
die genannte Schicht aus dem Material gemäß einem der Ansprüche 1 bis 14 zusammgesetzt ist, worin die aus einer Faserschicht gebildete Basisschicht so angeordnet ist, daß sie die äußere Seite bildet, die in Kontakt mit der Haut des Benutzers gelangt.

18. Absorbierbarer verwerfbarer Hygieneartikel wie ein Einlagen- bzw. Windelhöschen, umfassend elastifizierte Dichtungslängsflügel oder -barrieren, die über oder entlang den Längsrändern des absorbierenden Kissens an der inneren Deckschicht des genannten Einlagen- bzw. Windelhöschens fixiert sind,
dadurch **gekennzeichnet,** daß
die genannten Flügel aus dem Material gemäß einem der Ansprüche 1 bis 14 zusammengesetzt sind, worin die aus einer Faserschicht gebildete Basisschicht so angeordnet ist, daß sie die äußere Seite bildet, die in Kontakt mit der Haut des Benutzers gelangt.

## Claims

1. Coated nonwoven material, comprising a base layer formed from a nonwoven fabric made of fibrous material and at least one layer of a thermoplastic film applied to the base layer and comprising at least one ethylene polymer, characterized in that the thermoplastic film comprises at least one antiblocking agent, adheres uniformly only to the surface of the base layer and has a peel strength, determined according to the NFT 76-112 standard (condition: peel angle of 180°, pull speed of 200 mm/min.), of at most 98 × 10⁻³ kN/m.

2. Material according to Claim 1, characterized in that the peel strength is between 6 × 10⁻³ and 70 × 10⁻³ kN/m.

3. Material according to either of Claims 1 and 2, characterized in that the thermoplastic film has a thickness of at most 10 µm (microns).

4. Material according to Claim 3, characterized in that the thermoplastic film has a thickness ranging from 5 to 8 µm (microns).

5. Material according to any one of Claims 1 to 4, characterized in that the ethylene polymer is chosen from the group comprising copolymers of ethylene with at least one α-olefin having from 3 to 8 carbon atoms, ethylene/alkyl (meth)acrylate copolymers in which the alkyl group is linear or branched and has a carbon number ranging from 1 to 6, and copolymers of ethylene with at least one vinyl ester of a saturated carboxylic acid having from 2 to 6 carbon atoms.

6. Material according to Claim 5, characterized in that the ethylene polymer is chosen from ethylene/alkyl (meth)acrylate copolymers.

7. Material according to Claim 6, characterized in that the ethylene polymer is chosen from ethylene/methyl (meth)acrylate copolymers.

8. Material according to Claim 7, characterized in that the ethylene polymer is the ethylene/methyl acrylate copolymer.

9. Material according to any one of Claims 6 to 8, characterized in that the ethylene/alkyl (meth)acrylate copolymers comprise from 2.5% to 40% by weight of units derived from an alkyl (meth)acrylate.

10. Material according to any one of Claims 1 to 9, characterized in that the antiblocking agent is chosen from the group comprising mineral fillers, amides of unsaturated fatty acids having at least 8 carbon atoms and ethylene bis(amides) of unsaturated fatty acids having at least 8 carbon atoms.

11. Material according to Claim 10, characterized in that the antiblocking agent is chosen from the group comprising of silica, talc, zinc stearate, stearamide, oleamide, palmitamide, erucamide, myristamide, behenamide, ethylene bis(oleamide), ethylene bis(erucamide) and ethylene bis(stearamide).

12. Material according to 1s [sic] Claim 11, characterized in that the antiblocking agent is talc and/or ethylene bis(stearamide).

13. Material according to any one of Claims 1 to 12, characterized in that the nonwoven fabric made of fibrous material is manufactured by the spun bond and thermal bonding process from essentially continuous molecularly oriented filaments based on polypropylene (homopolymer) and has a basis weight of between 14 g/m² and 30 g/m².

14. Material according to Claim 13, characterized in that the polypropylene-based composition is modified by the addition of 0.8 to 1% by weight of an N-alkylamide of a fatty acid and in that the filaments have a diameter of between 1.8 and 2.2 dtex [sic].

15. Process for manufacturing a coated nonwoven material according to any one of Claims 1 to 14, which consists in:
- extruding a thermoplastic film (1);
- bringing the said thermoplastic film into contact with a web of nonwoven (4) ; and
- hauling off the web of nonwoven coated with the said thermoplastic film;
characterized in that the extruded thermoplastic film (1), having a temperature of at most 320°C and not yet solidified, is brought into contact with the web of nonwoven (4) by means of two rolls (5, 6) between which the pressure is at most 6 × 10⁵ Pa, the roll (5) in direct contact with the extruded thermoplastic film (1) being at a temperature of at most 25°C, and in that the web of nonwoven coated with the thermoplastic film is hauled off by a haul-off unit which has a speed of at most 300 m/min. and exerts a haul-off tension of at most 5 × 10⁵ Pa.

16. Disposable absorbent article of hygiene, such as a pair of nappy-pants or a pair of trainer pants, characterized in that the liquid-impermeable support sheet consists of the material according to any one of Claims 1 to 14, with the base layer formed from a nonwoven fabric placed towards the outside of the nappy-pants.

17. Disposable absorbent article of hygiene, such as a pair of nappy-pants, in which the inner covering sheet is provided with a central opening and with elastic elements which act in conjunction with the latter, characterized in that the said sheet consists of the material according to any one of Claims 1 to 14, with the base layer formed from a nonwoven fabric placed so as to form the external face which comes into contact with the wearer's skin.

18. Disposable absorbent article of hygiene, such as a pair of nappy-pants, comprising flaps or elasticated longitudinal sealing barriers fixed, on top of or along the longitudinal edges of the absorbent pad, to the inner covering sheet of the said nappy-pants, characterized in that the said flaps are made of the material according to any one of Claims 1 to 14, with the base layer formed from a nonwoven fabric placed so as to form the external face which comes into contact with the wearer's skin.
